# EUROPEAN PATENT SPECIFICATION

(11) **EP 1 895 036 B1**
(45) Date of publication and mention of the grant of the patent: **07.10.2009**
(21) Application number: 06017860.5
(22) Date of filing: 28.08.2006
(51) Int. Cl.: D04B 1/18, D04B 9/52, A61F 13/08

(54) **Knit construction for a medical compression garment**
Medizinische Kompressionskleidung
Vêtement de compression médical

(43) Date of publication of application: 05.03.2008
(73) Proprietor: Ganzoni Management AG, 8400 Winterthur (CH)
(72) Inventor: Ganzoni, Stefan, 4103 Bottmingen (CH); Berthéas, Alain, 42170 Saint-Just Saint-Rambert (FR); Fayolle, Christophe, 42600 Precieux (FR); Austernaud, Robert, 42400 St. Chamond (FR)
(74) Representative: Blum, Rudolf Emil

(56) References cited:
- FR-A1- 2 751 003
- FR-A1- 2 781 816
- FR-A1- 2 789 301
- GB-A- 1 219 734
- GB-A- 2 127 444
- JP-A- 2002 227 002
- JP-A- 2005 029 934

## Description

The present invention relates to a knit construction for a medical compression garment, a medical compression garment comprising such a knit construction and the use of a knitting machine for fabrication of a medical compression garment with a machine gauge of 16 to 32 needles per inch (630 to 1260 needles per metre) for manufacturing such a knit construction or such a medical compression garment.

Medical compression garments (abbreviated by MCG) a garments which are mainly made from elastic knitted fabric. Their aim is to provide a pressure or compressive force, respectively, to a human skin, especially to a limb such as an arm or a leg, for e.g. the treatment of venous diseases and lymphatic disorders. Medical compression garments are elastic garments that can, for example, be used to compress a leg below the knee, an entire leg, an arm, a hand and so on. They can be designed as stockings, socks, panties, arm sleeves, gloves etc.

Patients with diseases like the above-mentioned venous diseases and lymphatic disorders are usually required to apply some form of elastic compressive orthese during an entire day for a long period of their lifetime and it is therefore desirable to provide an orthese in the form of a garment, in particular in the form of a medical compression garment. Patients are generally more compliant with wearing the medical compression garment if it is comfortable to wear. Subsequently, the better the comfort of the used fabric of the garment, the better the treatment compliance can be obtained.

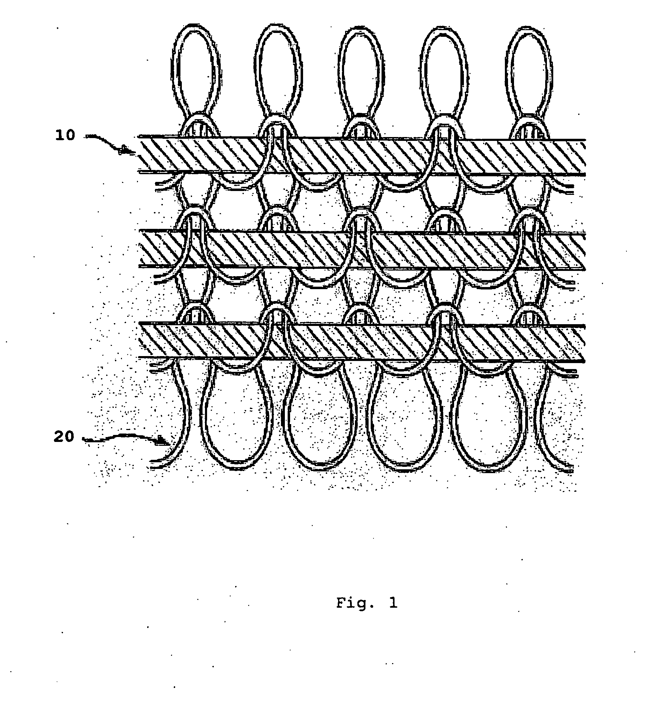

Medical compression garments and their fabrication must comply with normative requirements and specifications such as the German norm RAL-GZ 387 or the French norms NF G30-102b. These norms impose technical requirements for ensuring medical efficacy of the medical compression garments. Criteria to be checked and requirements to be met are the used materials and the physical performance i.e. the compressive effect of the medical compression garments. The requirements imposed on the fabric construction, that is the knitting pattern of the fabric of the medical compression garment, involve the so-called inlaid yarns 10 in combination with the so-called loop yarns 20 (confer Figure 1). The inlaid yarns 10 preferably comprise elastic material. The inlaid yarn 10 and the loop yarn 20 are specifically related to the compressive part of a medical compression garment.

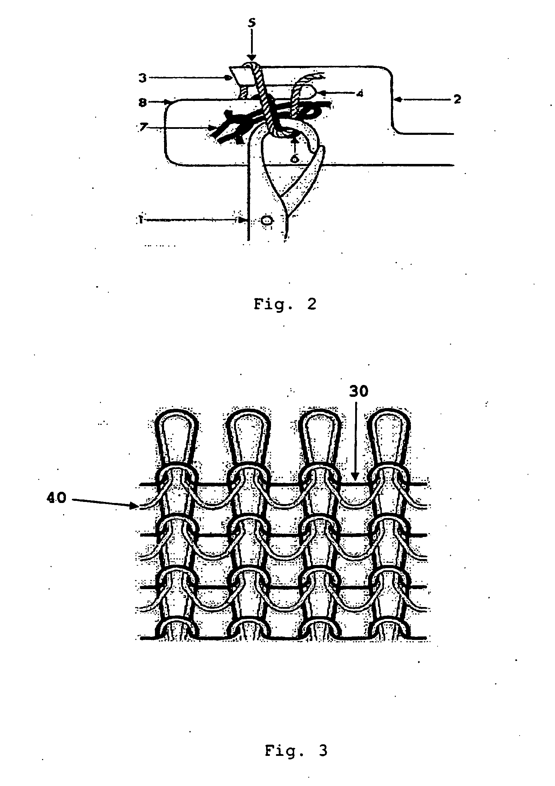

In some areas or parts of the medical compression garment a compressive force is not required. Such areas are for example a knee, a pantyhose, an insole and/or a toe area of the medical compression garment. Especially at these non-compressive areas or parts of the medical compression garment it is therefore possible to provide a soft touch on the inner side of the used fabric, the inner side being the side that rests against the skin of a patient when the medical compression garment is donned or worn. Such a soft touch on the inner side may for example be provided by the so-called terry-loop stitch (confer Figure 3). The technique to produce the terry-loop stitch pattern can, however, only be performed with the special knitting equipment shown in Figure 2.

The types of knitting machines which are able to manufacture medical compression garments that meet the above-mentioned norm requirements are today rather limited. Very few knitting machine manufacturers supply appropriate knitting machines that produce medical compression garments while meeting the above-mentioned norm requirements, in particular the norm requirements with regard to the inlaid yarns 10 in combination with the loop yarns 20. The limited number of types of appropriate knitting machines restricts therefore today's product development with respect to medical compression garments.

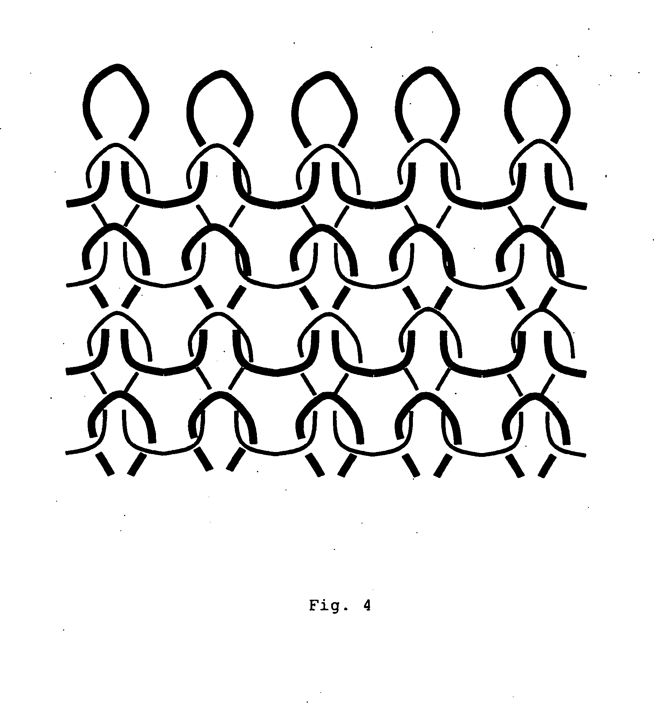

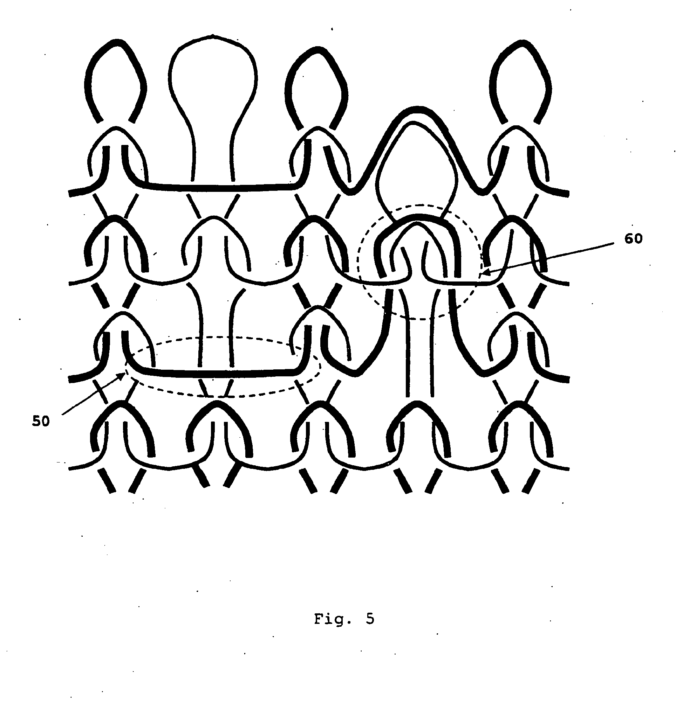

Today's knitting machines can for example perform the so-called plain stitch (see Figure 4), the so-called floated stitch 50 (see Figure 5) and the tuck stitch 60 (see Figure 5). According to the used knitting pattern, that is the used stitches, it is possible to obtain various visual and functional aspects/effects for the fabric and, hence, for the medical compression garment. The functional aspects are related to a patient's compliance factor i.e. the medical efficiency and the wear comfort of the medical compression garment. Today's knitting machines currently having machine gauges of 16 to 32 needles per inch (630 to 1260 needles per metre) are, however, not able to perform the terry-loop stitch

The wear comfort of the terry-loop pattern is mainly due to the thickness of the fabric caused by the bulky effect of the terry-loop stitch. The bulky aspect or effect of the terry-loop stitch or the terry-loop pattern, respectively, is obtained by the so-called terry yarn 40 which sticks out of the fabric by some length and is not held or retained by the employed ground yarn 30 (see Figure 3). Figure 2 shows a principle diagram of the fabrication of a terry-loop stitch. The bulky effect of the terry-loop stitch is obtained by employing a sinker 2. A ground fabric 7 is produced by grabbing a terry-loop yarn 5 and a ground yarn 6 by a needle 1. The ground yarn 6 is lead over a lower part 8 of the sinker 2. The terry-yarn 5 is lead over a sinker nip 3, wherein the sinker nip 3 is positioned above the lower part 8 of the sinker 2. The sinker throat 4 separates the sinker nip 3 from the lower part 8. The ground yarn 6 is lead though the sinker throat 4. In such a way it is assured that the terry-loop yarn 5 gains some extra length, leading to the bulky of the terry-loop stitch.

Patent application GB 2 127 444 A discloses a knit construction for therapeutic stockings that has a repeating six-course, two wale pattern in which the six courses include four courses comprising alternating knit
and tuck stitches and two courses comprising alternating knit and float stitches.

From patent document GB 1 219 734 A a knitted elastic fabric is known that is composed of a stretch yarn and a bare elastic yarn, wherein the elastic yarn is knit in every fourth wale and is floated across the three intervening wales to form floats. The elastic yarn may be incorporated in alternate courses or every third or fourth course, etc., as desired. Similarly, the elastic yarn may be knit in alternate wales or every third wale, or otherwise as desired.

Patent application JP 2002 227002 A discloses a knit construction in form of a stocking which is suitable for usage as a medical compression garment. Three stitches of each row appear to be formed as tuck stitches and one stitch appears to be formed as plain stitch.

Patent application FR 2 789 301 A discloses a support stocking that is knitted with a base yarn and a stretch yarn. The stretch yarn is knitted together with the base yarn in simple jersey stitches with one row of stitches on two. The base yarn is a multifilament yarn whose thickness is equal to or less than 50 dtex. The stretch yarn has a thickness, when stretched over the limp, of equal to or less than 50 dtex. The stretch yarn is a covered yarn, the core being of elastane. The mantle covering of the stretch yarn consists of seven filaments and has a thickness of 11 dtex.

In patent application JP 2005 029934 A a knitted fabric structure with slip preventing properties is described that is suitable for use as a medical compression garment. The knitted fabric structure comprises a rubber or polyurethane yarn and a fibre cross-knitting the rubber or polyurethane yarn. As fibre a composite fibre is used that consists of the rubber or polyurethane yarn, a covering yarn, a co-twisted yarn and an air-mixed yarn.

Patent application FR 2 781 816 A discloses a weft knitted fabric that has a knitted base structure A with ground stitches and additional rows of lock stitches with a stretch yarn on the weft, a second zone B formed within the base structure A with a medium holding action and/or a third zone C with a weak holding action. The inserted zone B is formed by the use of long stitches which replace the normal ground stitches and stretch lock stitches and doubled stitches. The weakest zone C is formed without a stretch weft, but with rows of ground stitches using an elastic synthetic filament, using normal stitches alternating with floating stitches, with stitch repeats over two to four rows. The zones B with a medium holding action are arranged in the knitted bandage product after it has been shaped to lie at a part of the body with a convex shape. The zones C with the weak holding action are placed where the body has concave shape and/or forms folds. The zones C with a weak holding action are formed by a thin synthetic stretch filament together with rows of normal stitches using a synthetic texturized filament.

It is an object of the invention to provide a knit construction for a medical compression garment that provides for an enhanced wear comfort and that can be manufactured with today's knitting machines in accordance with the above-mentioned norm requirements. Such knitting machines currently have machine gauges of 16 to 32 needles per inch (630 to 1260 needles per metre). It is a further object of the invention to provide a medical compression garment comprising such a knit construction and to provide for the use of a knitting machine with a machine gauge of 16 to 32 needles per inch (630 to 1260 needles per metre) for manufacturing such a knit construction or such a medical compression garment. The provided enhanced wear comfort preferably is similar to the wear comfort provided by the terry-loop pattern.

It is still a further object of the invention to provide a medical compression garment with a bulky touch, which is preferably similar to the touch of a terry-loop pattern, at the inner side of a medical compression garment and, in particular, at specific non-compressive parts of the medical compression garment such as for example a knee, a pantyhose, an insole or a toe area.

In order to implement these and still further objects of the invention, which will become more readily apparent as the description proceeds, a knit construction for medical compression garment is provided, that comprises stitches that are formed as plain stitch, tuck stitch or floated stitch. The plain stitch, the tuck stitch and the floated stitch can all be produced by existing knitting machines for medical compression garments while meeting the above-mentioned norm requirements. Tuck stitch patterns are made by having some needles knitting while other needles just have the yarn loops put into the needle hook. The needles in upper working position do not permanently knit, but an extra loop of yarn is laid over them with each pass of the carriage of the knitting machine. When these needles are returned to working position, all the loops on the needle knit in a single stitch resulting in the texture fabric. The tuck stitch 60 is depicted in Figure 5. The floated stitch 50 also comprises prises a loop of yarn which is not knitted in one column but just lies in front of the texture. Hence, tuck stitch 60 and floated stitch 50 provide for a bulky aspect which leads to an enhanced wear comfort. The plain stitch (confer Figure 4), the tuck stitch 60 and the floated stitch 50 are commonly available functions on existing knitting machines with machine gauge of 16 to 32 needles per inch (630 to 1260 needles per metre). Hence, by employing plain stitch, tuck stitch 60 and floated stitch 50 an effect similar to the terry-loop effect may be obtained with existing knitting machines while meeting the above-mentioned norm requirements. The effect or aspect obtained by a knit construction comprising stitches formed as plain stitch, tuck stitch or floated stitch may also be called pseudo terry-loop aspect, meaning that the functionality is essentially the same as the functionality of a terry-loop pattern.

The knit construction according to the invention preferably comprises at least four rows and four columns of stitches, wherein each row and each column comprises four stitches. The knit construction can, of course, comprise a multitude of four rows and four columns of stitches. One stitch of each odd row, that is for example the first row and the third row, is formed as a floated stitch, the remaining three stitches of each odd row being formed as plain stitches. The floated stitch of each odd row can be any stitch of the corresponding row. Three stitches of each even row, that is for example the second and the forth row, are formed as tuck stitches, the remaining stitch of each even row being formed as plain stitch. The three tuck stitches of each even row can be any three stitches of the corresponding row. By such a choice of stitch pattern, it is possible to increase the thickness and the softness of the knit construction providing a better wear comfort.

For further increasing the wear comfort, the odd rows, that is for example the first and the third row, are formed from single or double covered core yarns, wherein the core yarns preferably comprise elastic material and preferentially have a yarn count of 11 to 78 dtex. This leads to high elasticity and durability of the knit construction. As covering yarn a multifilament synthetic yarn and/or an artificial or natural staple yarn may be used, preferably having a yarn count of 33 to 110 dtex.

The even rows are preferably formed from composite yarns, in particular from composite air jet intermingled yarns. Such composite yarns are disclosed in the patent application FR 2 751 003 A1 . The composite yarn comprises an elastic yarn, a synthetic yarn, in particular a multifilament synthetic yarn comprising micro fibres, and an additional natural or artificial yarn, in particular a cotton yarn. The three different yarns can be intermingled by employing an air jet to form the composite yarn as disclosed in the patent application FR 2 751 003 A1. The material of the multifilament synthetic yarn of the composite yarn preferably comprises polyamide and the multifilament synthetic yarn preferentially has a yarn count of 33 to 110 dtex. The elastic yarn of the composite yarn preferably has a yarn count of 11 to 78 dtex. The additional natural or artificial yarn of the composite yarn is preferably a cellulosic yarn, preferentially having a yarn count of 100 to 400 dtex.

A medical compression garment according to the invention comprises a knit construction according to the invention as described above as a first knit construction and a second knit construction, wherein the stitches of the second knit construction are formed by employing an inlaid yarn, preferably an elastic inlaid yarn, and a loop yarn. The first knit construction preferentially forms a non-compressive part of the medical compression garment and the second knit construction preferably forms a compressive part of the medical compression garment. The non-compressive part may form a pantyhose, a knee, an insole and/or a toe area of the medical compression garment.

By providing a knit construction according to the invention at the non-compressive part of a medical compression garment a soft touch can be provided at the inner side of a medical compression garment leading to an increased wear comfort at the non-compressive parts such as, for example, the pantyhose, the knee, the toe and/or the insole area of the medical compression garment and therefore to an enhancement of the overall wear comfort. By organising the status of each stitch as plain, tuck or floated stitch and by using appropriate yarns with adequate yarn counts, in particular the composite yarn, it is therefore possible to obtain a similar effect as can be obtained by employing terry-loop stitches. Hence, a pseudo terry-loop effect can be obtained by using existing knitting machines for medical compression garments complying with normative requirements. Available knitting machines for fabrication of medical compression garments with a machine gauge of 16 to 32 needles per inch (630 to 1260 needles per metre) can advantageously be used for manufacturing a knit construction according to the invention or a medical compression garment according to the invention.

Further advantageous features and applications of the invention can be found in the dependent claims as well as in the following description of the drawings illustrating the invention. In the drawings like reference signs designate the same or similar parts throughout the several figures of which:
Figure 1 shows an exemplary stitch pattern of a compressive part of a medical compression garment,
Figure 2 illustrates the manufacturing of a terry-loop stitch,
Figure 3 shows a terry-loop stitch pattern,
Figure 4 shows a plain stitch pattern,
Figure 5 shows a stitch pattern comprising a tuck stitch and a floated stitch,
Figure 6 illustrates the assignment of symbols to various stitch types,
Figure 7 shows a preferred embodiment of a knit construction according to the invention,
Figure 8 depicts in principle the composition of a composite yarn.

Figure 1 displays a knit construction that forms a compressive part of a medical compression garment. Inlaid yarns 10, which are preferably elastic yarns, are combined with loop yarns 20. The loop yarn 20 can be in front of or behind the inlaid yarn 10. The shown knit construction imposes pressure upon a limb when the corresponding medical compression garment comprising the shown knit construction is donned on the limb or worn.

Figures 2 and 3 relate to the terry-loop stitch. Figure 2 illustrates the procedure for manufacturing the terry-loop stitch shown in Figure 3. The manufacturing procedure of Figure 2 has been described above. For the terry-loop stitch a terry-loop yarn 40 and a ground yarn 30 are employed. The terry-loop yarn 40 is not held by the ground yarn 30. Hence, an extra length of the terry-loop yarn 40 is provided which leads to the bulky effect of the terry-loop stitch and to increased wear comfort. The terry-loop stitch can, however, not be performed by existing knitting machines for medical compression garments having a gauge of 16 to 32 needles per inch (630 to 1260 needles per metre).

Figures 4 and 5 show various stitch types that can be performed by existing knitting machines. Figure 4 depicts the plain stitch. Figure 5 depicts the floated stitch 50 and the tuck stitch 60 as described above.

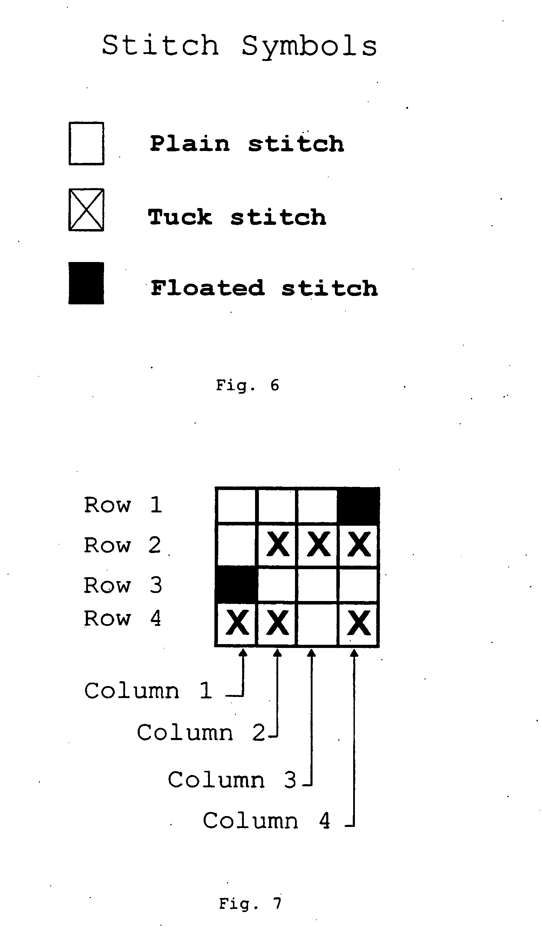

Figure 7 shows a preferred embodiment of a knit construction according to the invention comprising four rows and four columns, wherein each row or each column, respectively, consists of four stitches. The preferred embodiment shown in Figure 7 aims at creating an especially bulky surface for the inner side of a medical compression garment that comprises the shown knit construction. According to Figure 6, each stitch is assigned a certain symbol for demonstration purposes. The plain stitch is assigned a white square, the tuck stitch is assigned a square with a cross, and the floated stitch is assigned a black square. The expression "square" includes rectangles.

In the preferred embodiment of Figure 7 one stitch of row 1 is formed as floated stitch and three stitches of row 1 are formed as plain stitches, wherein the floated stitch can be anyone of the four stitches of row 1 and the three plain stitches can be the remaining three stitches. Row 3 as an odd row is formed equivalently to row 1, the floated stitch being any of the four stitches of row 3. Row 2 and row 4 each comprise three tuck stitches, the location of the tuck stitches not being mandatory, that is the three tuck stitches can be any three stitches of each of the even rows. The remaining stitch of row 2 and row 4 is formed as plain stitch. Other combinations of the plain stitch, the tuck stitch and the floated stitch are possible.

By combination of the plain stitch, the tuck stitch and the floated stitch in particular as depicted in Figure 7, a knit construction is obtained that provides enhanced wear comfort, leading to a pseudo terry-loop effect in softness and thickness of the knit construction. The knit construction can form a non-comprehensive part of a medical compression garment with the extra loops of the tuck stitches and the floated stitches (confer Figure 5) preferably being on the inner side of the medical compression garment. The inner side of the medical compression garment is defined as the side which is in contact with the skin of a limb of a patient when the medical compression garment is donned or worn.

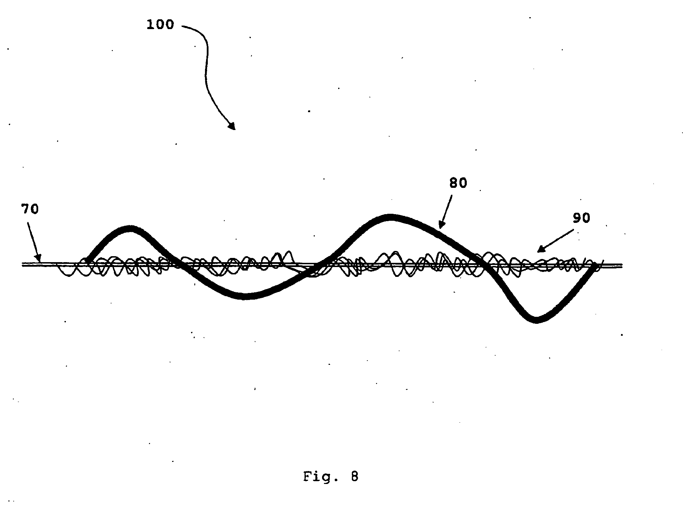

Rows 2 and 4 are preferably knitted by using a composite yarn as described in the patent application FR 2 751 003 A1. Figure 8 depicts such a composite yarn 100 including an elastic yarn 70, a multifilament synthetic yarn 90 and a third additional natural or artificial yarn 80, in particular a cotton yarn. The multifilament synthetic yarn 90 preferably consists of micro fibres and is intermingled together with the natural or artificial yarn 80 around the elastic yarn 70.

The non-compressive part of the medical compression garment can for example form a pantyhose, a knee, an insole and/or a toe area of the medical compression garment.

It is to be understood that while certain embodiments of the present invention have been illustrated and described herein, it is not to be limited to the specific embodiments described and shown.

## Claims

1. A knit construction for a medical compression garment comprising at least four rows and four columns of stitches that are formed as plain stitch, tuck stitch (60) or floated stitch (50), **characterized in that** one stitch of each odd row is formed as floated stitch (50) and three stitches of each odd row are formed as plain stitches, and three stitches of each even row are formed as tuck stitches (60) and one stitch of each even row is formed as plain stitch.

2. A knit construction according to claim 1, wherein the odd rows are formed from single or double covered core yarns.

3. A knit construction according to claim 2, wherein the core yarns comprise elastic material and preferably have a yarn count of 11 to 78 dtex.

4. A knit construction according to claim 2 or 3, wherein a multifilament synthetic yarn and/or an artificial or natural staple yarn is used as covering yarn, preferably having a yarn count of 33 to 110 dtex.

5. A knit construction according to one of the preceding claims, wherein the even rows are formed from composite yarns (100), in particular from composite air jet intermingled yarns.

6. A knit construction according to claim 5, wherein each composite yarn (100) comprises an elastic yarn (70), a multifilament synthetic yarn (90) and an additional natural or artificial yarn (80).

7. A knit construction according to claim 6, wherein the material of the multifilament synthetic yarn (90) comprises polyamide and the multifilament synthetic yarn (90) preferably has a yarn count of 33 to 110 dtex.

8. A knit construction according to claim 6 or 7, wherein the additional natural or artificial yarn (80) is a cellulosic yarn, preferably having a yarn count of 100 to 400 dtex.

9. A medical compression garment comprising a knit construction according to one of the preceding claims as a first knit construction and a second knit construction, the stitches of the second knit construction being formed by employing an inlaid yarn (10) and a loop yarn (20).

10. A medical compression garment according to claim 9, wherein the first knit construction forms a non-compressive part of the medical compression garment and the second knit construction forms a compressive part of the medical compression garment.

11. A medical compression garment according to claim 10, wherein the non-compressive part forms a pantyhose, a knee, an insole and/or a toe area of the medical compression garment.

12. Use of a knitting machine for fabrication of a medical compression garment with a machine gauge of 16. to 32 needles per inch (630 to 1260 needles per metre) for manufacturing a knit construction according to one of the claims 1 to 8 or a medical compression garment according to one of the claims 9 to 11.

## Patentansprüche

1. Eine Strickkonstruktion für ein medizinisches Kompressionskleidungsstück umfassend wenigstens vier Reihen und für Spalten aus Stichen, die als Einfachstich, als Fanghenkel (60) oder als Flottierstich (50) ausgeführt sind, **dadurch gekennzeichnet, dass** ein Stich einer jeden ungeraden Reihe als Flottierstich (50) ausgeführt ist und dass drei Stiche einer jeden ungeraden Reihe als Einfachstiche ausgeführt sind, und dass drei Stiche einer jeden geraden Reihe als Fanghenkel (60) ausgeführt sind und dass ein Stich einer jeden geraden Reihe als Einfachstich ausgeführt ist.

2. Eine Strickkonstruktion nach Anspruch 1, wobei die ungeraden Reihen aus einfach oder doppelt umhüllten Kerngarnen geformt sind.

3. Eine Strickkonstruktion nach Anspruch 2, wobei die Kerngarne elastisches Material umfassen und vorzugsweise eine Garnstärke von 11 bis 78 dtex haben.

4. Eine Strickkonstruktion nach Anspruch 2 oder 3, wobei ein synthetisches Multifilamentgarn und/oder ein künstliches oder natürliches Stapelfasergarn als umhüllendes Garn eingesetzt ist, das vorzugsweise eine Garnstärke von 33 bis 110 dtex hat.

5. Eine Strickkonstruktion nach einem der vorhergehenden Ansprüche, wobei die geraden Reihen von zusammengesetzten Garnen (100) gebildet sind, insbesondere von zusammengesetzten, mittels Luftstrahl vermischten Garnen.

6. Eine Strickkonstruktion nach Anspruch 5, wobei jedes zusammengesetzte Garn (100) ein elastisches Garn (70), ein synthetisches Multifilamentgarn (90) und ein zusätzliches natürliches oder künstliches Garn (80) umfasst.

7. Eine Strickkonstruktion nach Anspruch 6, wobei das Material des synthetischen Multifilamentgarns (90) Polyamid umfasst und das synthetische Multifilamentgarn vorzugsweise eine Garnstärke von 33 bis 110 dtex hat.

8. Eine Strickkonstruktion nach Anspruch 6 oder 7, wobei das zusätzliche natürliche oder künstliche Garn (80) ein cellulosehaltiges Garn ist, das vorzugsweise eine Garnstärke von 100 bis 400 dtex hat.

9. Eine medizinisches Kompressionskleidungsstück umfassend eine Strickkonstruktion gemäss einem der vorhergehenden Ansprüche als erste Strickkonstruktion und eine zweite Strickkonstruktion, wobei die Stiche der zweiten Strickkonstruktion durch das Einsetzen eines eingelegten Garns (10) und eines Schlaufengarns (20) geformt sind.

10. Ein medizinisches Kompressionskleidungsstück nach Anspruch 9, wobei die erste Strickkonstruktion einen nicht-zusammenpressenden Teil des medizinischen Kompressionskleidungsstücks bildet und die zweite Strickkonstruktion einen zusammenpressenden Teil des medizinischen Kompressionskleidungsstücks bildet.

11. Ein medizinisches Kompressionskleidungsstück nach Anspruch 10, wobei der nicht-zusammenpressende Teil eine Strumpfhose, ein Knie, eine Innensohle und/oder einen Zehenbereich des medizinischen Kompressionskleidungsstücks bildet.

12. Verwendung einer Strickmaschine zur Fertigung eines medizinischen Kompressionskleidungsstücks mit einem Maschinenmass von 16 bis 32 Nadeln pro Inch (630 bis 1260 Nadeln pro Meter) zum Herstellen einer Strickkonstruktion gemäss einem der Ansprüche 1 bis 8 oder eines medizinischen Kompressionskleidungsstücks gemäss einem der Ansprüche 9 bis 11.

## Revendications

1. Structure à mailles pour un vêtement de compression médical comportant au moins quatre rangées et quatre colonnes de mailles formées de mailles ordinaires, mailles de cueillage (60) ou flottées (50), **caractérisée en ce qu'**une maille de chaque rangée impaire est une maille flottée (50) et trois mailles de chaque rangée impaire sont des mailles ordinaires, et trois mailles de chaque rangée paire sont des mailles de cueillage (60) et une maille de chaque rangée paire est une maille ordinaire.

2. Structure à mailles selon la revendication 1, dans laquelle les rangées impaires sont formées de fil guipé simple ou double.

3. Structure à mailles selon la revendication 2, dans laquelle les fils guipés contiennent une matière élastique et ont de préférence un numéro de fil compris entre 11 et 78 dtex.

4. Structure à mailles selon la revendication 2 ou 3, dans laquelle un fil multi-filament synthétique et/ou un fil discontinu artificiel ou naturel est utilisé comme fil de guipage, de préférence ayant un numéro de fil de 33 à 110 dtex.

5. Structure à mailles selon la revendication selon l'une des revendications précédentes, dans laquelle les rangées paires sont formées de fil composite (100) notamment de fils composites entremêlés au jet d'air.

6. Structure à mailles selon la revendication 5, dans laquelle chaque fil composite (100) comporte un fil élastique (70), un fil multi-filament synthétique (90) et un fil supplémentaire (80) naturel ou artificiel.

7. Structure à mailles selon la revendication 6, dans laquelle la matière du fil multi-filament synthétique (90) comprend un polyamide et le fil multi-filament synthétique (90 )a de préférence un numéro de fil de 33 à 110 dtex.

8. Structure à mailles selon la revendication 6 ou 7, dans laquelle le fil supplémentaire (80) naturel ou artificiel (80) est un fil cellulosique, de préférence ayant un numéro de fil de 100 à 400 dtex.

9. Vêtement de compression médical comportant une structure à mailles selon l'une des revendications précédentes comme première structure à mailles et une seconde structure à mailles, les mailles de la seconde structure étant formées en employant un fil tramé (10) et un fil bouclé (20).

10. Vêtement de compression médical selon la revendication 9, dans lequel la première structure à mailles forme une partie non compressive du vêtement de compression médical et la seconde structure à mailles forme une partie compressive du vêtement de compression médical.

11. Vêtement de compression médical selon la revendication 10, dans lequel la partie non compressive forme un bas-culotte, un genou, une semelle et/ou une surface de pointe du vêtement de compression médical.

12. Utilisation d'une machine à tricoter pour la fabrication d'un vêtement de compression médical avec une jauge de machine de 16 à 32 aiguilles au pouce (630 à 1260 aiguilles au mètre) pour fabriquer une structure à mailles selon l'une des revendications 1 à 8 ou un vêtement de compression médical selon l'une des revendications 9 à 11.
